# EUROPEAN PATENT SPECIFICATION

(11) **EP 3 501 401 B1**
(45) Date of publication and mention of the grant of the patent: **09.12.2020**
(21) Application number: 17306914.7
(22) Date of filing: 22.12.2017
(51) Int. Cl.: A61B 7/04, A61B 7/02, A61B 5/021, A61B 5/022, A61B 5/0402

(54) **APPARATUS AND METHOD FOR AUSCULTATION**
VORRICHTUNG UND VERFAHREN ZUR AUSKULTATION
APPAREIL ET PROCÉDÉ D'AUSCULTATION

(43) Date of publication of application: 26.06.2019
(73) Proprietor: Withings, 92130 Issy-les-Moulineaux (FR)
(72) Inventor: CAMPO, David, 92130 Issy-les-Moulineaux (FR)
(74) Representative: Plasseraud IP

(56) References cited:
- US-A- 4 672 976
- US-A- 5 010 890
- US-A1- 2003 088 385

## Description

### FIELD OF THE DISCLOSURE

The present disclosure relates to apparatuses and methods for auscultation.

### BACKGROUND OF THE DISCLOSURE

Heart auscultation is of great interest since some of heart disorders can be detected non-invasively by analysing characteristic sounds emitted by the heart cyclic operation.

There are known auscultation devices with one stethoscope head and with a filtering function seeking to eliminate background noises from the heart sounds.

However, there remains a need to provide improved methods and systems to enhance analysis of heart sounds and detection of heart conditions.

A prior art attempt is disclosed in document US4672976 disclosing an apparatus comprising a pair of sound sensing devices for being placed in an operative position at predetermined locations on the precordial region of a patient and hold in place by strap means.

### SUMMARY OF THE DISCLOSURE

There is disclosed an apparatus according to claim 1 comprising a cuff configured to be worn around a upper arm of a user, a first acoustic sensor (S1) comprised by the cuff and configured to receive sounds through a side of the chest of the user, and a second acoustic sensor (S2) configured to receive sounds and to be movable with regard to the cuff and configured to be placed at a front side of the user's chest and used in conjunction with the first sensor.

There is provided a two-sensor apparatus that can be used by a user himself/herself, the first sensor being placed against the side of the chest at a repeatable location. For most people this is the left side of the chest but a small minority are dextrocardiac which means the heart is on the right side of the chest. The second sensor is placed by hand of the user. Thereby, the apparatus receives signals representative of heart sounds sensed a two different locations at the user's chest and it will be possible to use these two signals to improve analysis of heart sounds.

It should be noted that the above apparatus can be used in a medical facility or at home, or in any environment, for self-auscultation either independently or with help of e.g. telemedicine services. The user can be a patient or an individual taking care of his/her health. The apparatus can be used for both diagnosis of illnesses and preventive healthcare. The first sensor is mounted on the cuff.

The use of two distinct stethoscope heads for heart auscultation notably allows to distinguish very short sounds which can be indicative of some suspected medical condition in on or more heart valves.

Further, one of the following features can be used alone or in combination.

The first acoustic sensor may be a first stethoscope head. This first stethoscope head may be used for auscultation. This first sensor may be provided in the arm cuff and therefore, it is easy for the user to correctly place the first sensor by placing the cuff around his/her upper arm, and then placing the arm against his/her chest.

The first acoustic sensor may be a second stethoscope head. This second stethoscope head may be used for auscultation. This second sensor is movable and can be handled easily and conveniently by one hand of the user.

According to an example embodiment, the first sensor (S1) may be fixedly mounted in the cuff. This first sensor may be housed in the arm cuff and therefore, it is easy and straightforward for the user to correctly place the first sensor by placing the cuff around his/her upper arm.

According to an example embodiment, the second sensor (S2) may be detachably mounted in the cuff. Whereby, when the apparatus is to be stowed, the second sensor can be attached to the cuff in a convenient manner. When in use, the second sensor is detached to be moved around.

According to an example embodiment, the arm cuff may comprise at least a pneumatic bladder for blood pressure measurement.

The apparatus can house circuity for a blood pressure measurement function.

In such case, the apparatus can perform a blood pressure measurement function together with an auscultation function. Whereby, monitoring of bio parameters can be enhanced.

According to an example embodiment, the second sensor and the cuff may be connected with a wired connection. A very reliable connection is thereby provided, insensitive to possible electromagnetic pollution or jammers. Wire shielding can be provided to reject extraneous electric noises. Privacy of signals is also ensured.

According to an example embodiment, the second sensor and the cuff may be wirelessly connected. This is a very convenient and user-friendly solution, the second sensor can be moved around freely without taking care of any physical connection. This configuration also allows to place the second sensor under clothing very easily since there is no physical connection between the second sensor and the cuff.

According to an example embodiment, the apparatus can be connected to a display device such as a smartphone, a tablet or a computer. Thereby, instructions, feedback, results of analysis, and possibly other notices can be given to the user. Also, user profile and personal history can be handled through the display device.

According to an example embodiment, the display device is configured to provide guidance on positioning the second sensor. It is therefore possible to guide the user such that the second sensor is positioned right in the interval between two ribs. This leads to improved signal-to-noise ratio and improvement of the quality of the signal coming from the second sensor.

According to an example embodiment, the apparatus may further comprise a control circuitry which comprises a noise cancelling circuitry which is configured to reduce noise from signals received from at least one of the first or second sensors. The noise cancelling circuitry may use signals from the first and/or signals from the second sensors. The control unit is able to compare signals from the first and second sensor and to eliminate partially or totally extrinsic noises, for example common mode noises, to output a denoised signal. Thereby it is possible to improve the quality and accuracy of signals indicative of sounds emitted by the heart of a user or patient.

According to an example embodiment, the apparatus the may be configured to be connected to one or more remote server(s), directly or through the display device. Therefore, the apparatus can be used in connection with telemedicine services. Analysis of a possible medical condition can be therefore performed by trained medical personnel, and/or with the help of a medical expert system.

According to an example embodiment, the apparatus can be configured to receive an ECG signal (ECG stands for electrocardiogram). Timings of heart sounds can be correlated to electrical signals coming from the heart. According to an example embodiment, an ECG function can be integrated in the apparatus.

According to an example embodiment, there may be provided two or more electrodes arranged either at an internal wall of the cuff and/or at the first sensor and/or at the second sensor. Thereby, mechatronic integration is improved.

According to an example embodiment, the apparatus can comprise a housing arranged adjacent to the cuff, the housing enclosing a control unit. Protection and strength are provided by a such housing.

According to an example embodiment, the apparatus can comprise a vibrator, for giving a haptic feedback to the user. Thereby, the user can be notified accurately when to stop moving the second sensor S2.

According to one aspect of the present disclosure, it is disclosed a method according to claim 13 comprising :
**/a/**- receiving signals from a first acoustic sensor (S1) attached to the external wall of a cuff worn around an upper arm of a user (75), such that, the first sensor (S1) is placed against the side of the user's chest, so as to receive sounds through the side of the user's chest;
**/b/-** receiving signals from a second acoustic sensor (S2) movable with regard to the cuff and placed at a front side of the user's chest,
**/c/**- analysing signals sensed by the first sensor and by the second sensor.

There may additionally be provided :
**/d/-** analysing the signals sensed by the first and second sensors for providing correction instructions to correct the placement of the second sensor (S2) thereby providing guidance for positioning the second sensor.

In particular, such correction instructions and guidance can help the user for positioning the second sensor at one of the prescribed heart auscultation positions (PI, P2, P3, P4, P5) at a front side of the user's chest.

Whereby, the first sensor is at a steady location, with a generally even signal level, whereas by contrast, while the second sensor is moved, the signal level received at the second sensor is affected by the presence of the ribs. Reference given by the first sensor provides guidance to help the user to place the second sensor properly at one of the prescribed positions.

According to one particular option, the method may further comprise:
sending first set of data to a remote server and receiving a second set of data from the remote server. The remote server may be part of a telemedicine service. Thereby, telemedicine can be involved to provide either correction instructions for placement of the second sensor and/or to provide diagnosis.

According to one particular option, the method may further comprise:
**/e1/**- signalling to the user that a pre-defined position for the second sensor is reached,
**/e2/-** processing the signals of the first sensor and/or the second sensor with a noise cancelling algorithm. Thereby, effective signal analysis starts after correct placement of first and second sensors and analysis is carried out with a stable configuration.

According to one particular option, the method may further comprise:
**/d2/-** providing correction instructions graphically on the display device coupled to the auscultation apparatus. Thereby, notice and guidance is given in an intuitive manner.

According to one particular option, the method may further comprise:
**/d3/-** providing feedback to notify proper position (for example by way of visual feedback, and/or audio feedback, and/or haptic feedback). Thereby, the user knows accurately when to stop moving the second sensor S2.

According to one particular option, the method may further comprise:
**/e/-** carrying out analysis of signals at a telemedicine service and sending back health information such as a diagnosis. Thereby, the apparatus can be used at home by an individual without the presence of any medical personnel and can be supported by remote telemedicine service(s) to get back health information such as a diagnosis.

### BRIEF DESCRIPTION OF THE DRAWINGS

Other features and advantages of the disclosure appear from the following detailed description of one of its embodiments, given by way of non-limiting example, and with reference to the accompanying drawings, in which:
- Figure 1 illustrates a general overview of an apparatus according an example embodiment in a use configuration,
- Figure 2 shows a diagrammatic sectional view of a cuff of the apparatus in place on the left arm of the user, and adjacent to the chest, and second sensor at the front of the chest,
- Figure 3 shows an illustrative block diagram of the apparatus according to a first example embodiment,
- Figure 4 shows a timeplot illustrating an example of the noise cancelling feature,
- Figure 5 shows a diagrammatic view of a chest skeleton, heart and locations of auscultation
- Figure 6 shows a timeplot illustrating the method at a larger timescale, when placing the second sensor,
- Figure 7 illustrates a general flowchart of data processing,
- Figure 8 shows an illustrative block diagram of a variant of the apparatus according to a second embodiment, having a blood pressure sensing function, and optionally a ECG function,
- Figure 9 shows a general timeplot illustrating the method, at the heartbeat timescale,
- Figure 10 shows a timeplot illustrating a detection of aortic stenosis,
- Figure 11 illustrates a general overview of the disclosed method,
- Figures 12A and 12B illustrate a detachable sensor.

### DETAILED DESCRIPTION OF THE DISCLOSURE

In the figures, the same references denote identical or similar elements. For clarity purposes, some parts are represented intentionally not at scale with regard to other parts. Also, some parts of timeplots can be represented intentionally not at scale.

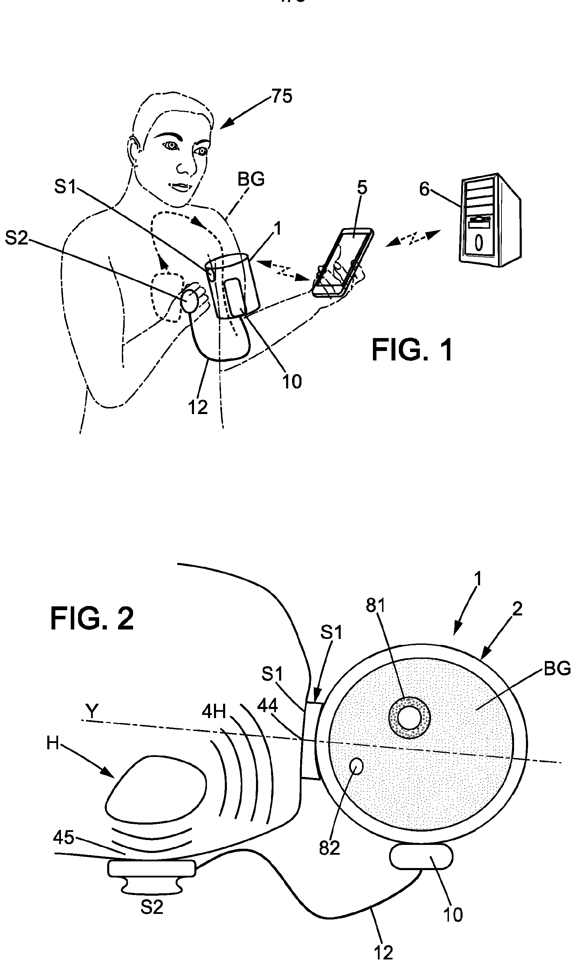

As shown in Figures 1 and 2, an apparatus **1** according to an example embodiment comprises a cuff **2,** a first sensor **S1** and a second sensor **S2.** The first sensor **S1** can be an acoustic sensor. The second sensor **S2** can be an acoustic sensor.

In an example embodiment, the first sensor **S1** is a first stethoscope head.

In an example embodiment, the second sensor **S2** is a second stethoscope head.

The apparatus **1** is an auscultation apparatus. In an example embodiment, the apparatus **1** is configured for heart auscultation.

The cuff **2** is formed as an arm band to be worn around an upper arm of a user **75.** As illustrated, the cuff is wrapped around the upper left arm of the user **75.** There is provided a housing **10** for housing a control unit that will be discussed later.

In one example of an embodiment, the cuff **2** is an arm band with clipping means and/or serrating means to be wrapped tightly and steadily around the upper arm.

In another example of embodiment, the cuff **2** may carry additional function(s) like blood pressure monitoring and optionally electrocardiogram (ECG) function.

The left arm **BG** of the user includes a bone named the humerus **81,** muscles (not shown), and the brachial artery **82.** The cuff **2,** when wrapped around the arm **BG,** has a general cylindrical shape with a reference axis substantially coinciding with the arm axis.

The first sensor **S1** is comprised by the cuff, whereas the second sensor **S2** is configured to be movable with regard to the cuff.

By the clause *"comprised by the cuff",* it shall be understood the first sensor can be mounted in the cuff or placed adjacent to the cuff itself as non-limiting examples.

By the clause *"mounted in the cuff",* it shall be understood the second sensor can be for example received and housed in a recess of the cuff in a detachable manner; the first sensor can be attached to the cuff, in a fixed manner. Other ways to mechanically associate the first sensor or the second sensor to the cuff can be envisioned.

The first sensor **S1** is attached to the external wall of the cuff. According to one example the first sensor **S1** is fixedly mounted in the cuff in a predetermined position, without possibility to move the first sensor **S1** with regard to the cuff. In such case, once the user has positioned the cuff correctly, the position of first sensor is correct.

According to another example, the first sensor **S1** can be placed at two or three predetermined positions, according to the type/gender of user or according to the size of the user's arm.

The first sensor **S1** has a sound receiving face **44** oriented away from the cuff axis (i.e. away from the arm). In the shown example, the first sensor is slightly projecting outwards; however alternately, the sensor can be fully integrated in the cuff without significant protrusion.

In use, the first sensor **S1** is placed against the side of the chest (left side for most people) at a repeatable location, and the second sensor **S2** is placed by hand of the user (here by the right hand). The height along the arm can be determined with reference to the biceps muscle, or with reference to the elbow joint.

In the shown example, the user holds a display device **5** in the left hand. In the shown example the display device 5 is a smartphone. The purpose of the display device will be discussed later.

The user **75** may be a patient or any individual wishing to monitor one or more of his/her bio-parameters. The apparatus can be used for both diagnosis of illnesses and preventive healthcare.

The apparatus can be used in medical facility or at home, or in any environment, for self-auscultation either independently or with help of medical personnel and/or telemedicine services.

In the shown example, the second sensor is coupled via a wire **12** to the cuff **2.** More precisely the wire is connected at one of its end to the control unit housing **10** and at the other end to the second sensor such as second stethoscope head. The length of the wire may be comprised between 25 cm and 60 cm.

The second sensor **S2** is detachably mounted in the cuff. When in use, the second sensor is detached from a stowed position to be moved around. When the apparatus is to be stowed, the second sensor can be attached to the cuff at the stowed position.

The stowed position can be defined as a position along the external wall of the cuff, where there may be provided a recess for receiving and maintaining there the second sensor S2.

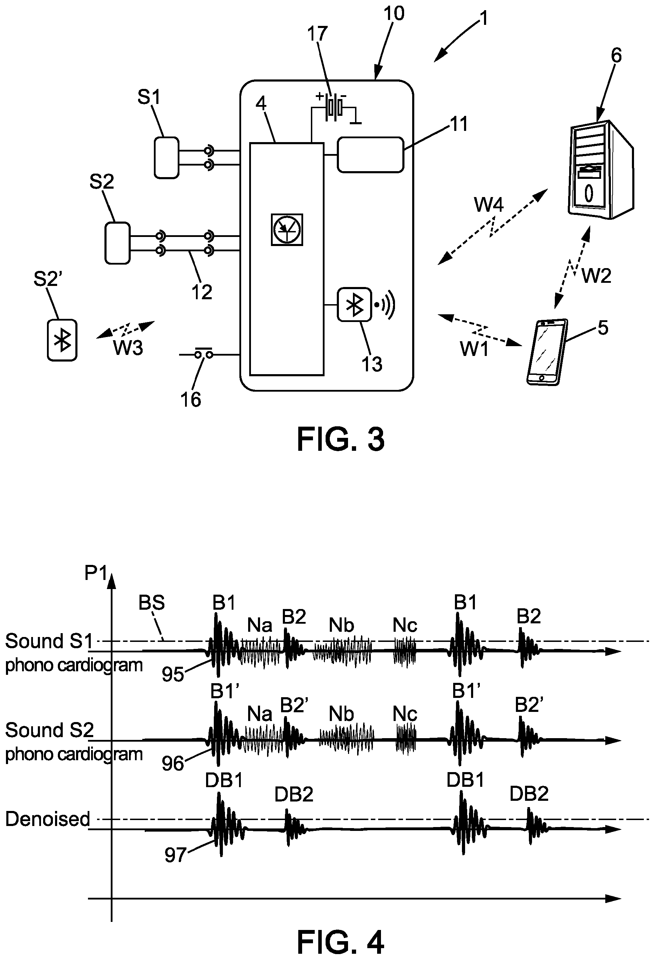

According to a variant, instead of the wire, a wireless link **W3** is used between the second sensor **S2'** (Figure 3) and the control unit **4.**

The apparatus **1** has a wireless communication capability **W1** to exchange data with a display device 5 such as a smartphone or other mobile device like a tablet or a laptop.

Such display device **5** may in turn exchange data with a remote entity like a remote server **6** more generally any resource accessible via a network connection, for example via the Internet, through a data link **W2** (wireless + internet). According to another configuration, there is provided a direct communication **W4** between the apparatus **1** and the remote server **6.**

Cyclic operation of the heart **H** generates sound waves **4H** which propagate in all directions across tissues.

The first sensor **S1** which is interposed between the left side of the chest and the arm equipped with the cuff has a sound receiving face **44** configured to receive the sound waves. The first sensor **S1** can be positioned on bare skin, or can be positioned on a piece of clothing. Interposition of clothing does not significantly decrease the sensed signal and signal quality is sufficient even if the first sensor is applied on a piece of underwear, shirt, T shirt, or the like.

The second sensor **S2** is movable with regard to the cuff, and is configured to be positioned at the front side of the chest. The positions of auscultation for the second sensor **S2** will be discussed below. Under this condition, the second sensor **S2** has a sound receiving face **45** placed against the chest and configured to receive sound waves from the heart **H.** The second sensor **S2** can be positioned on bare skin, or can be positioned on a piece of clothing; indeed an interposition of a clothing does not significantly decrease much the sensed signal and signal quality is sufficient even if the second sensor **S2** is applied on a piece of underwear, shirt, T shirt, or the like.

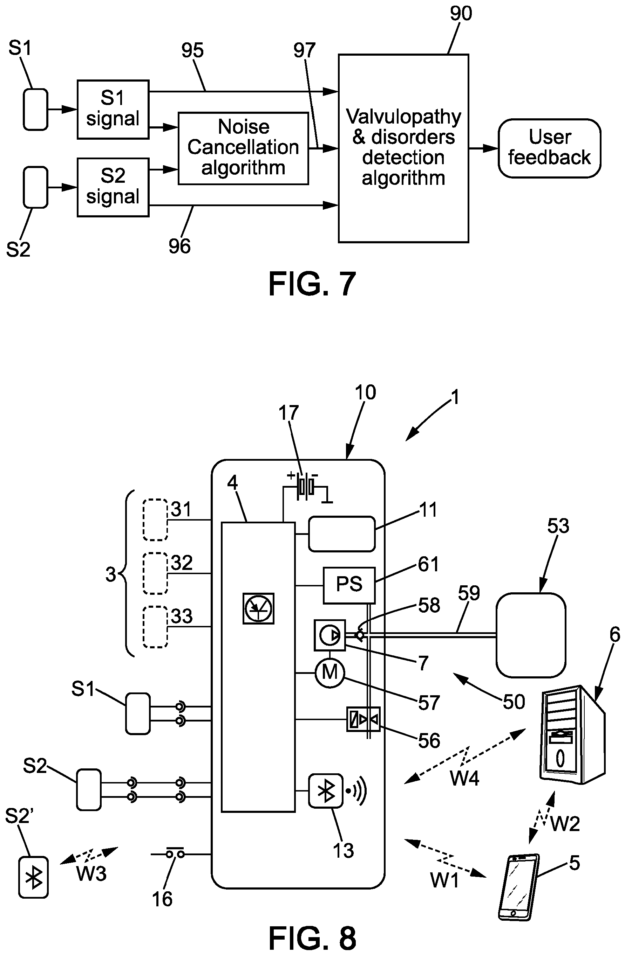

As shown in Figures 3 and 8, the housing **10** comprises a control unit **4.**

The **control unit 4** receives signals from the first sensor **S1** and the second sensor **S2.** There may be provided a battery **17** serving as power source. There may be provided the local display **11.** There is provided a wireless transceiver **13** (Bluetooth™, WiFi, or any similar solutions). There is provided a switch **16.**

The control unit **4** comprises a control circuitry which may comprise a noise cancelling circuitry.

According to a first embodiment (Figure 3, Figure 4), the apparatus **1** can be used for auscultation. The apparatus **1** can be used for auscultation of an animal or a human individual.

The example use cases described above concern heart auscultation. However, other use cases are possible. Lung auscultation can also be carried out with the apparatus **1.**

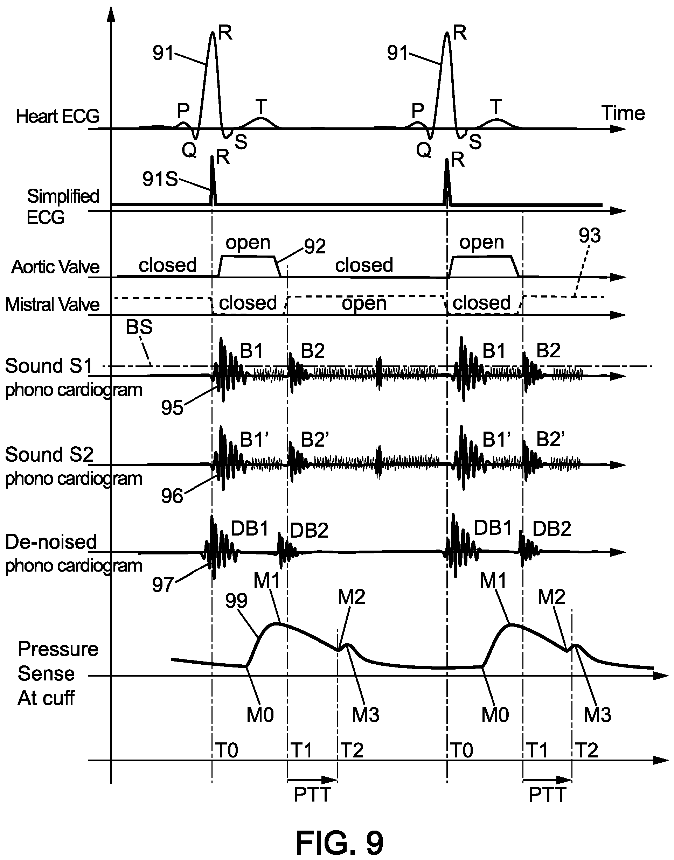

According to a second embodiment (Figure 8, Figure 9), the apparatus 1 has additional features. The apparatus 1 may be provided with a blood pressure sensing function and/or an ECG function. These two additional functions will be described later.

Returning to the first example embodiment as depicted at Figures 3,4,7, the control unit **4** is configured to analyze a first signal **95** received from the first sensor S1 and a second signal **96** received from the second sensor **S2.**

Phonocardiograms representing the first and second signals **95, 96** are shown at Figure 4. A phonocardiogram (PCG) is a graphic representation of heart sounds.

### Noise cancellation algorithm

The sounds picked up at the first sensor S1 and the second sensor S2 can be affected by various noises **Na, Nb, Nc** at Figure 4.

Such noises **Na, Nb, Nc** for heart auscultation can come from lungs, bowels, movement of the user, cloth rubbing, or even from remote devices like air conditioning, conversations nearby, telephone ringing, door banging, aircraft passing by, drilling works in the building, etc..

The control unit is configured to use an algorithm to suppress noises and keep intrinsic sounds of the heart.

Subtracting signals picked up at the first sensor **S1** from the second sensor **S2** allows to eliminate or reduce common mode signals which affect both sensors similarly.

Further, the noise cancellation algorithm can be based on a Wavelet analysis.

According to a variant the noise cancellation algorithm can be based on a FFT analysis.

According to one option, digital filtering can be used, to eliminate or reduce highfrequency signal components.

A noise cancellation algorithm can be applied to first signal alone, or to second signal alone. A noise cancellation algorithm can be applied in a combined manner to first and second signals.

At the output of a noise cancellation block described at Figure 7, a third signal **97** is available. In one example, the third signal **97** is a low noise signal, since most the noise has been removed from the first signal and/or the second signal by the noise cancellation block.

As shown at Figures 4 and 9, the phonocardiogram representing the first signal **95** exhibits two characteristics sounds; the first sound **B1** corresponds to the closing of the mitral valve, the second sound **B2** corresponds to the closing of the aortic valve. The phonocardiogram representing the second signal **96** exhibits similar characteristics sounds; **B1'** corresponds to the closing of the mitral valve and tricuspid valve, **B2'** corresponds to the closing of the aortic valve and pulmonary valve.

DB1 and DB2 correspond to denoised sounds, result of the above-mentioned algorithms.

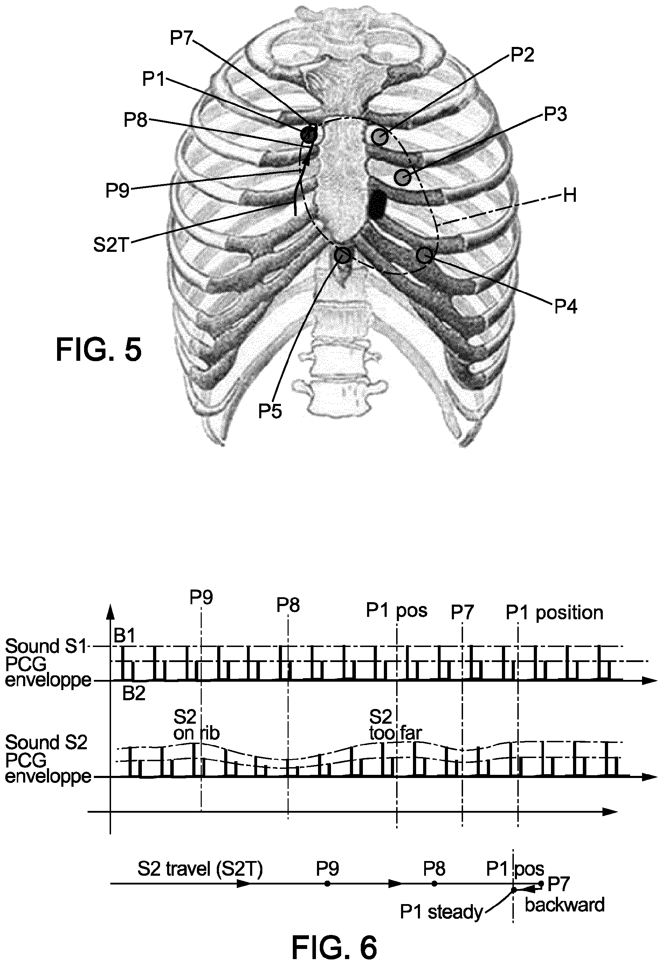

Referring now to Figure 5, medical practice defines some prescribed heart auscultation positions **P1, P2, P3, P4, P5,** as illustrated at Figure 5.
**P1** is known as aortic area : right second inter costal space
**P2** is known as pulmonic area : left second inter costal space
**P3** is known as "Erb's point" : left third inter costal space
**P4** is known as mitral area : left fifth inter costal space,
**P5** is known as tricuspid area : left lower sternal border

In an example embodiment, the apparatus **1** provides correction instructions to correct the placement of the second sensor **S2** at one of the prescribed heart auscultation positions (**P1, P2, P3, P4, P5**) at a front side of the user's chest, as illustrated at Figure 6.

When the user moves the second sensor **S2** along the travel **S2T,** the output of the first sensor S1 is not affected by movement of the second sensor S2 and therefore the envelope of the signal transduced by first sensor **S1** is flat. By contrast, the envelope of the signal transduced by second sensor **S2** fluctuates according to the presence or not of a rib or sternum interposed between the second sensor **S2** and deep tissues (e.g. heart).

As illustrated, at position **P9,** the amplitude envelope is high, whereas by contrast at position **P8,** the amplitude envelope is low. The target position here is **P1.**

The analysis of the envelope of the signal transduced by second sensor **S2** allows the control unit **4** to give a notice to the user either to go on moving or to stop moving or to go back.

**P7** position is a bit too far since the amplitude envelope has decreased.

When it is determined that the position **P1** has been reached, the user is notified for example with a message "stop moving" and "keep steady".

Figure 7 summarises the data flow, wherein functional blocks are shown, with functionalities partially or totally performed by the control circuitry of the control unit. There is provided an analysis block **90** which comprises a valvulopathy and disorders detection algorithm. Firstly, the first signal **95** is analysed individually; secondly the second signal **96** is analysed individually, and the third signal **97** issued by the noise cancellation algorithm is also analysed in conjunction with the two first ones.

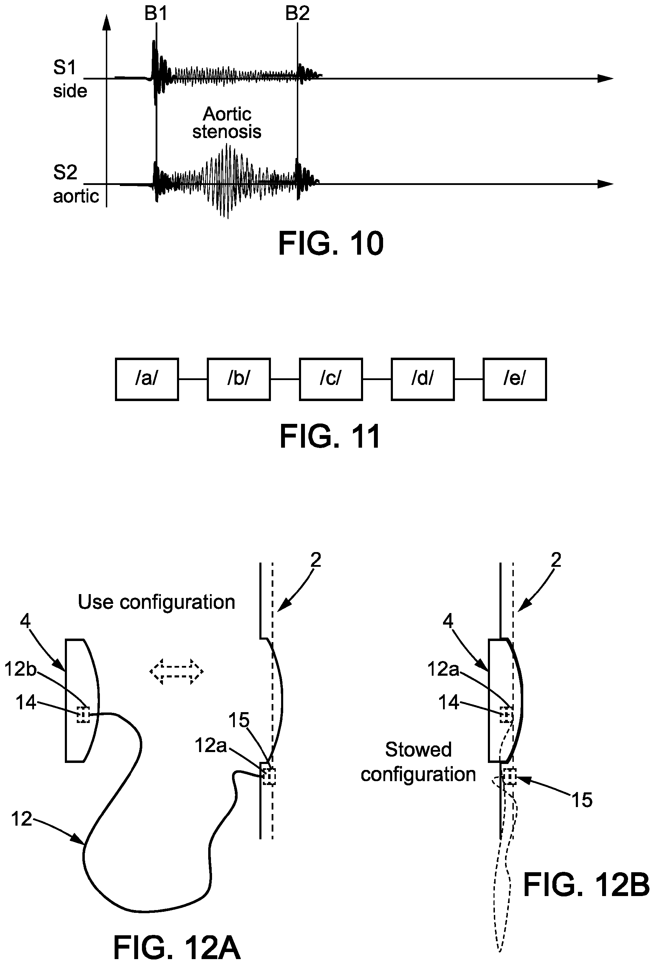

The algorithm in the analysis block **90** outputs a report with unexpected noises, such as noises being characteristic of a valve disorder. One example of a heart disorder, aortic stenosis, is given in Figure 10, without any intended limitation.

In one embodiment, the algorithm in the analysis block **90** is fully implemented locally in the control unit **4.**

In a variant embodiment, the algorithm in the analysis block **90** is implemented partly locally in the control unit **4** and partly in the display device **5.**

In another variant embodiment, the algorithm in the analysis block **90** is implemented partly locally in the control unit **4** and partly in the remote server **6.**

In another variant embodiment, the algorithm in the analysis block **90** is implemented partly locally in the control unit **4,** partly in the display device **5** and partly in the remote server **6.**

In another variant embodiment, the analysis block is implemented partly in the display device **5** and partly in the remote server **6.**

The second sensor **S2** can be of a wired type or of a wireless type (S2').

As illustrated at Figure 12A, in the case of the wired type, together with the cuff **2,** there may be provided the above-mentioned cable **12.** At one end of this cable, there is provided a connector **12a** counterpart of the base connector **15;** at the other end, there is provided a connector **12b** counterpart of the second sensor connector **14.** The cable 12 can comprise two wires. More wires are not excluded.

The cable **12** can be detached for stowage. In another embodiment, the cable can remain and be wrapped or stowed against the cuff (see Figure 12B).

According to an extended functionality variant of the device, the device comprises a blood pressure sensing function at the cuff.

There is provided a pneumatic unit **50** with an inflatable bladder **53** partly or totally around along the cuff.

The pneumatic unit **50** comprises at least a pump **7** which may be driven by an electric motor **57,** a release valve **56,** and a pressure sensor **61.**

According to a particular embodiment, the inflatable bladder **53** is connected to the pneumatic unit by an integrated pneumatic connector, or otherwise via a tube **59.**

The pneumatic unit **50** may optionally comprise a check valve **58.** The release valve 56 may be an on/off valve or a proportional valve.

The housing **10** comprises a switch **16.** The switch **16** can be a press switch, a capacitor switch or a touch switch. The switch can have an on/off function, a 'start cycle' function, without excluding auxiliary functions using short press and long press feature.

There can be provided also a tap actuation function using an embedded accelerometer.

The user may start a blood pressure measurement, after having installed the armband, by actuating the switch **16.**

The device is powered by a battery **17.** The battery is for example a rechargeable battery. The battery can be a lithium ion battery.

For a blood pressure measurement session, the control unit **4** is configured to first inflate the inflatable bladder **53** until blood flow is greatly reduced by the pressure exerted on the arm. During inflation, the analysis of the evolution of pressure signals allows to infer the systolic pressure and the diastolic pressure. The controller is configured to then progressively deflate the inflatable bladder **53.** The progressive reinstatement of the blood pressure waves is also analyzed by the control unit **4** to infer the systolic pressure and the diastolic pressure, in confirmation or replacement of values deduced during the inflation phase.

Also, by using one or two of the acoustic sensor(s), it is possible to calculate the pulse transit time (PTT), as explained later.

According to an extended functionality variant of the device, the device comprises a ECG function.

In this configuration, there are provided ECG electrodes **31,32,33** collectively denoted **3.** In an embodiment, at least two of the ECG electrodes are arranged in the inwardly facing wall of the cuff. There may be another one arranged at the external surface of the housing 10. In variant configurations, one of the ECG electrodes is arranged at the first sensor **S1**, and one of the ECG electrodes is arranged at the second sensor **S2.**

As illustrated at Figure 9, waves accompany each heartbeat, and the heartbeat generates electrical waves that propagate through the body at a first speed and the heartbeat generates a pressure wave in artery network that propagates through the vasculature at a second slower speed.

Immediately after the ventricular contraction, the pressure wave leaves the heart and aorta, passes through the subclavian artery, to the brachial artery along the path **P.**

The ECG electrodes **31,32,33** capture electrical signals which pass to an amplifier and a filtering circuit within the control unit **4.** For example, a filtering circuit is provided before the signal is digitized and entered into the microcontroller.

Within the control unit **4,** the ECG signals are processed with an analog-to-digital converter to form the digitized ECG waveform and then recorded together with the time of occurrence, namely instant **T0**. ECG waveform includes a characteristic part, i.e. "QRS waveform" or "QRS complex".

A simplified ECG curve **91S** is shown at the second curve of Figure 9.

The acoustic waves are also band-pass filtered and amplified, for example after digitization. For example, a band-pass filter with cutting frequencies [0,5 Hz - 1kHz] is applied, either in the analog font end before digitization or applied to the digitized acoustic signal.

A QRS waveform is shown in the ECG signal **91** on timeplot 'Heart ECG' at Figure 9. Instant **T0** corresponds in the illustrative embodiment to R apex, but another timing characteristic can be taken alternatively.

Aortic valve open/close state is also shown, signal **92.**

Mitral valve open/close state is also shown, signal **93.**

Just before aortic valve opening, the mitral valve closes. This produces a particular sound which is reflected in the first significant sound **B1** as shown on signal **95.**

The second sound **B2** corresponds to the closing of the aortic valve at time **T1**.

It should be noted that the three phonocardiograms representing the first signal **95,** the second signal **96,** and the third signal **97** are similar to what has been explained before regarding Figure 4, and thus not repeated here.

Further, after closing mitral valve and opening aortic valve, ventricular volume decreases as blood is ejected to the aorta. At the same time ventricular pressure exhibits a rounded apex.

The pressure curve **99** exhibits three characteristics apexes. The first apex **M1** is a maximum apex; the second apex **M2** is a minimum local apex; the third apex **M3** is a maximum local apex.

Besides **M0** is the minimum value, just before the rise which is a consequence of the arrival of the pressure pulse at the arm.

The second apex **M2** is a marker corresponding to arrival of the effect of the closure of aortic valve at the brachial artery within the arm band. **M2** occurs at time **T2.** Time interval **T2-T1** is referred to as pulse transit time **PTT.**

It should be noted that when using the **ECG** signal (either complete ECG signal **91** or simplified signal **91S**), the control unit **4** benefits from an improved time reference, instead of relying only on the first sound **B1**.

It should be noted that measuring systolic pressure and diastolic pressure, and optionally pulse transit time **PTT,** provides complementary information besides the phonocardiograms that can be utilized by an expert system or medical staff, to provide a more complete and more reliable diagnosis.

Pressure curve analysis can reinforce detection of aortic valve disorder. An oscillometric signal normally has an expected characteristic shape, and an aortic valve disorder can be suspected or determined whenever the characteristic of an oscillometric signal does not meet that characteristic shape.

As illustrated at Figure 11, the disclosed method may comprise the steps **/a/, /b/, /c/, /d/** as mentioned earlier in the summary of the disclosure. Further, the disclosed method may comprise the step **/e/** as mentioned earlier in the summary of the disclosure.

According to one embodiment step **/d/** may comprise the steps **/d2/** and/or **/d3/** as mentioned earlier in the summary of the disclosure. According to one embodiment step **/e/** may comprise the steps **/e1/** and **/e2/** as mentioned earlier in the summary of the disclosure.

## Claims

1. An apparatus (1) comprising:
a cuff configured to be worn around an upper arm of a user (75);
a first acoustic sensor (S1) attached to the external wall of the cuff and having a sensitive side (41) oriented away from the axis of the cuff, such that in use the first sensor (S1) is placed against the side of the user's chest, so as to receive sounds through the side of the user's chest; and
a second acoustic sensor (S2) configured to be used in conjunction with the first sensor and movable with regard to the cuff, such that in use it is placed at a front side of the user's chest so as to receive sounds through the front side of the user's chest.

2. An apparatus according to claim 1, in which the first sensor (S1) is fixedly mounted in the cuff.

3. An apparatus according to any of the claims 1 to 2, in which the second sensor (S2) is detachably mounted in the cuff.

4. An apparatus according to any of the claims 1 to 3, in which the cuff comprises a pneumatic bladder for blood pressure measurement.

5. An apparatus according to any of the claims 1 to 4, in which the second sensor (S2) and the cuff are connected with a wired connection (12).

6. An apparatus according to any of the claims 1 to 5, in which the second sensor (S2') and the cuff are wirelessly connected.

7. An apparatus according to any of the claims 1 to 6, in which the apparatus is configured to be connected to a display device (5).

8. An apparatus according to claim 7, the display device (5) being configured to provide guidance on positioning the second sensor (S2).

9. An apparatus according to any of the claims 1 to 8, further comprising a control circuitry which comprises a noise cancelling circuitry which is configured to reduce noise from signals received from at least one of the first or second sensors.

10. An apparatus according to any of the claims 1 to 9, configured to be connected to one or more remote server(s) (6).

11. An apparatus according to any of the claims 1 to 10, wherein the apparatus is configured to receive an ECG signal.

12. An apparatus according to any of the claims 1 to 10, provided with two or more electrodes (31,32,33) arranged at an internal wall of the cuff and/or at the first sensor S1 and/or at the second sensor S2.

13. A method comprising :
**/a/-** receiving acoustic signals from a first acoustic sensor (S1) attached to the external wall of a cuff worn around an upper arm of a user (75), the first acoustic sensor (S1) having a sensitive side oriented away from the cuff axis, such that, the first sensor (S1) is placed against the side of the user's chest, so as to receive sounds through the side of the user's chest;
**/b/-** receiving acoustic signals from a second acoustic sensor (S2) configured to be movable with regard to the cuff and placed at a front side of the user's chest, so as to receive sounds through the fron side of the user's chest;
**/c/-** using acoustic signals sensed by the first sensor in conjunction with and simultaneously with acoustic signals sensed by the second sensor,
**/d/-** analysing the signals sensed by the first and second sensors (S1, S2) for providing correction instructions to correct the placement of the second sensor (S2) thereby providing guidance for positioning the second sensor (S2).

14. A method according to claim 13, further comprising :
**/e1/-** signalling to the user that a pre-defined position (P1, P2, P3, P4, P5) for the second sensor is reached; and
**/e2/-** processing the signals of the first sensor S1 and the second sensor S2 with a noise cancelling algorithm.

15. A method according to claim 13, further comprising :
**/d2/-** providing correction instructions graphically on the display device coupled to the auscultation apparatus, and/or
**/d3/-** providing feedback to notify proper position, by way of visual feedback, and/or audio feedback, and/or haptic feedback.

## Patentansprüche

1. Vorrichtung (1), die aufweist:
eine Manschette, die konfiguriert ist, um um einem Oberarm eines Benutzers (75) getragen zu werden;
einen ersten akustischen Sensor (S1), der an der Außenwand der Manschette befestigt ist und eine empfindliche Seite (41) hat, die von der Achse der Manschette weg orientiert ist, so dass der erste Sensor (S1) in Verwendung gegen die Brustseite des Benutzers angeordnet wird, um durch die Brustseite des Benutzers Töne zu empfangen; und
einen zweiten akustischen Sensor (S2), der konfiguriert ist, um in Verbindung mit dem ersten Sensor verwendet zu werden, und der in Bezug auf die Manschette beweglich ist, so dass er in Verwendung auf einer Vorderseite der Brust des Benutzers angeordnet ist, um durch die Vorderseite der Brust des Benutzers Töne zu empfangen.

2. Vorrichtung nach Anspruch 1, wobei der erste Sensor (S1) fest in der Manschette montiert ist.

3. Vorrichtung nach einem der Ansprüche 1 bis 2, wobei der zweite Sensor (S2) abnehmbar in der Manschette montiert ist.

4. Vorrichtung nach einem der Ansprüche 1 bis 3, wobei die Manschette eine pneumatische Blase für die Blutdruckmessung aufweist.

5. Vorrichtung nach einem der Ansprüche 1 bis 4, wobei der zweite Sensor (S2) und die Manschette über eine verdrahtete Verbindung (12) verbunden sind.

6. Vorrichtung nach einem der Ansprüche 1 bis 5, wobei der zweite Sensor (S2') und die Manschette drahtlos verbunden sind.

7. Vorrichtung nach einem der Ansprüche 1 bis 6, wobei die Vorrichtung konfiguriert ist, um mit einer Anzeigevorrichtung (5) verbunden zu werden.

8. Vorrichtung nach Anspruch 7, wobei die Anzeigevorrichtung (5) konfiguriert ist, um eine Führung für die Positionierung des zweiten Sensors (S2) bereitzustellen.

9. Vorrichtung nach einem der Ansprüche 1 bis 8, die ferner eine Steuerschaltungsanordnung aufweist, die eine Rauschunterdrückungsschaltungsanordnung aufweist, die konfiguriert ist, um Rauschsignale aus Signalen, die von wenigstens einem des ersten und zweiten Sensors empfangen werden, zu verringern.

10. Vorrichtung nach einem der Ansprüche 1 bis 9, die konfiguriert ist, um mit einem oder mehreren entfernten Server(n) (6) verbunden zu werden.

11. Vorrichtung nach einem der Ansprüche 1 bis 10, wobei die Vorrichtung konfiguriert ist, um ein EKG-Signal zu empfangen.

12. Vorrichtung nach einem der Ansprüche 1 bis 10, der mit zwei oder mehr Elektroden (31, 32, 33) versehen ist, die an einer Innenwand der Manschette und/oder an dem ersten Sensor S1 und/oder an dem zweiten Sensor S2 eingerichtet sind.

13. Verfahren, das aufweist:
/a/- Empfangen akustischer Signale von einem ersten akustischen Sensor (S1), der an der Außenwand einer Manschette befestigt ist, die um einen Oberarm eines Benutzers (75) getragen wird, wobei der erste akustische Sensor (S1) eine empfindliche Seite hat, die von der Manschettenachse weg orientiert ist, so dass der erste Sensor (S1) gegen die Brustseite des Benutzers angeordnet ist, um durch die Brustseite des Benutzers Töne zu empfangen;
/b/- Empfangen akustischer Signale von einem zweiten akustischen Sensor (S2), der konfiguriert ist, um in Bezug auf die Manschette beweglich zu sein und auf einer Vorderseite der Brust des Benutzers angeordnet zu werden, um durch die Vorderseite der Brust des Benutzers Töne zu empfangen;
/c/- Verwenden akustischer Signale, die von dem ersten Sensor in Verbindung mit und gleichzeitig mit akustischen Signalen, die von dem zweiten Sensor abgetastet werden, abgetastet werden,
/d/- Analysieren der Signale, die von dem ersten und zweiten Sensor (S1, S2) abgetastet werden, um Korrekturanweisungen bereitzustellen, um die Anordnung des zweiten Sensors (S2) zu korrigieren, wodurch eine Führung zur Positionierung des zweiten Sensors (S2) bereitgestellt wird.

14. Verfahren nach Anspruch 13, das ferner aufweist:
/e1/- Signalisieren, dass eine vordefinierte Position (P1, P2, P3, P4, P5) für den zweiten Sensor erreicht ist, an den Benutzer; und
/e2/- Verarbeiten der Signale des ersten Sensors S1 und des zweiten Sensors S2 mit einem Rauschunterdrückungsalgorithmus.

15. Verfahren nach Anspruch 13, das ferner aufweist:
/d2/- graphisches Bereitstellen von Korrekturanweisungen auf der Anzeigevorrichtung, die mit der Auskultationsvorrichtung gekoppelt ist, und/oder
/d3/- Bereitstellen einer Rückmeldung, um mittels visueller Rückmeldung und/oder hörbarer Rückmeldung und/oder haptischer Rückmeldung die richtige Position zu melden.

## Revendications

1. Appareil (1) comprenant :
un brassard configuré pour être porté autour d'un avant-bras d'un utilisateur (75) ;
un premier capteur acoustique (S1) fixé à la paroi externe du brassard et ayant un côté sensible (41) orienté à l'opposé de l'axe du brassard, de telle sorte que durant l'utilisation le premier capteur (S1) est placé contre le côté du thorax de l'utilisateur, de manière à recevoir des sons à travers le côté du thorax de l'utilisateur ; et
un second capteur acoustique (S2) configuré pour être utilisé conjointement au premier capteur et déplaçable par rapport au brassard, de telle sorte que durant l'utilisation il est placé au niveau d'un côté avant du thorax de l'utilisateur de manière à recevoir des sons à travers le côté avant du thorax de l'utilisateur.

2. Appareil selon la revendication 1, dans lequel le premier capteur (S1) est monté de manière fixe dans le brassard.

3. Appareil selon l'une quelconque des revendications 1 et 2, dans lequel le second capteur (S2) est monté de manière amovible dans le brassard.

4. Appareil selon l'une quelconque des revendications 1 à 3, dans lequel le brassard comprend une vessie pneumatique pour la mesure de la pression artérielle.

5. Appareil selon l'une quelconque des revendications 1 à 4, dans lequel le second capteur (S2) et le brassard sont reliés par une liaison filaire (12).

6. Appareil selon l'une quelconque des revendications 1 à 5, dans lequel le second capteur (S2') et le brassard sont reliés sans fil.

7. Appareil selon l'une quelconque des revendications 1 à 6, dans lequel l'appareil est configuré pour être relié à un dispositif d'affichage (5).

8. Appareil selon la revendication 7, le dispositif d'affichage (5) étant configuré pour fournir une orientation lors du positionnement du second capteur (S2).

9. Appareil selon l'une quelconque des revendications 1 à 8, comprenant en outre un circuit de commande qui comprend un circuit d'annulation du bruit qui est configuré pour réduire le bruit de signaux reçus d'au moins l'un des premier ou second capteurs.

10. Appareil selon l'une quelconque des revendications 1 à 9, configuré pour être relié à un ou plusieurs serveurs distants (6).

11. Appareil selon l'une quelconque des revendications 1 à 10, dans lequel l'appareil est configuré pour recevoir un signal d'ECG.

12. Appareil selon l'une quelconque des revendications 1 à 10, doté de deux électrodes (31, 32, 33) ou plus agencées au niveau d'une paroi interne du brassard et/ou au niveau du premier capteur S1 et/ou au niveau du second capteur S2.

13. Procédé comprenant :
/a/ - la réception de signaux acoustiques d'un premier capteur acoustique (S1) fixé à la paroi externe d'un brassard porté autour d'un avant-bras d'un utilisateur (75), le premier capteur acoustique (S1) ayant un côté sensible orienté à l'opposé de l'axe de brassard, de telle sorte que, le premier capteur (S1) est placé contre le côté du thorax de l'utilisateur, de manière à recevoir des sons à travers le côté du thorax de l'utilisateur ;
/b/ - la réception de signaux acoustiques d'un second capteur acoustique (S2) configuré pour être mobile par rapport au brassard et placé au niveau d'un côté avant du thorax de l'utilisateur, de manière à recevoir des sons par l'intermédiaire du côté avant du thorax de l'utilisateur ;
/c/ - l'utilisation de signaux acoustiques détectés par le premier capteur conjointement et simultanément aux signaux acoustiques détectés par le second capteur,
/d/ - l'analyse des signaux détectés par les premier et second capteurs (S1, S2) pour fournir des instructions de correction pour corriger le placement du second capteur (S2) fournissant ainsi une orientation pour le positionnement du second capteur (S2).

14. Procédé selon la revendication 13, comprenant en outre :
/e1/ - la signalisation à l'utilisateur qu'une position prédéfinie (P1, P2, P3, P4, P5) pour le second capteur est atteinte ; et
/e2/ - le traitement des signaux du premier capteur S1 et du second capteur S2 avec un algorithme d'annulation du bruit.

15. Procédé selon la revendication 13, comprenant en outre :
/d2/ - la fourniture d'instructions de correction graphiquement sur le dispositif d'affichage couplé à l'appareil d'auscultation, et/ou
/d3/ - la fourniture d'un retour pour notifier la position correcte, au moyen d'un retour visuel, et/ou d'un retour audio, et/ou d'un retour haptique.
